# EUROPEAN PATENT SPECIFICATION

(11) **EP 1 234 175 B1**
(45) Date of publication and mention of the grant of the patent: **05.03.2008**
(21) Application number: 00948137.5
(22) Date of filing: 24.07.2000
(51) Int. Cl.: G01N 33/48

(54) **IMPROVEMENTS IN AND RELATING TO FORENSIC INVESTIGATIONS**
VERBESSERUNGEN IN HINBLICK AUF FORENSISCHE UNTERSUCHUNGEN
INVESTIGATIONS JUDICIAIRES AMELIOREES

(30) Priority: 23.07.1999 GB 9917308
(43) Date of publication of application: 28.08.2002
(73) Proprietor: Forensic Science Service Ltd, London EC3N 2AA (GB)
(72) Inventor: NOAKE, Penelope Jane, The Forensic Science Service, Birmingham B5 6QQ (GB); GRIMES, Eileen Ann, The Forensic Science Service, Birmingham B5 6QQ (GB); URQUHART, Andrew James, The Forensic Science Serv, Birmingham B5 6QQ (GB); LOWE, Alexandra Louise, The Forensic Science Serv, Birmingham B5 6QQ (GB)
(74) Representative: Pawlyn, Anthony Neil
(86) International application number: PCT/GB2000/002792
(87) International publication number: WO 2001/007908

(56) References cited:
- WO-A-97/44485
- FRANDBERG PER-ANDERS ET AL: "Human pigmentation phenotype: A point mutation generates nonfunctional MSH receptor." BIOCHEMICAL AND BIOPHYSICAL RESEARCH COMMUNICATIONS, vol. 245, no. 2, 17 April 1998 (1998-04-17), pages 490-492, XP001026335 ISSN: 0006-291X
- SMITH RACHEL ET AL: "Melanocortin 1 receptor variants in an Irish population." JOURNAL OF INVESTIGATIVE DERMATOLOGY, vol. 111, no. 1, July 1998 (1998-07), pages 119-122, XP001027724 ISSN: 0022-202X
- DATABASE BIOSIS [Online] BIOSCIENCES INFORMATION SERVICE, PHILADELPHIA, PA, US; 1995 VALVERDE PALOMA ET AL: "Variants of the melanocyte-stimulating hormone receptor gene are associated with red hair and fair skin in humans." Database accession no. PREV199698562770 XP002188623 & NATURE GENETICS, vol. 11, no. 3, 1995, pages 328-330, ISSN: 1061-4036
- DATABASE BIOSIS [Online] BIOSCIENCES INFORMATION SERVICE, PHILADELPHIA, PA, US; 1996 VALVERDE PALOMA ET AL: "The Asp84Glu variant of the melanocortin 1 receptor (MC1R) is associated with melanoma." Database accession no. PREV199699249107 XP002188624 & HUMAN MOLECULAR GENETICS, vol. 5, no. 10, 1996, pages 1663-1666, ISSN: 0964-6906
- P. VALVERDE ET AL: "Variants of the melanocyte stimulating hormone receptor gene are associated with red hair and fair skin in humans" NATURE GENETICS, vol. 11, no. 3, 1995, pages 328-330,

## Description

This invention concerns improvements in and relating to forensic investigations, particularly, but not exclusively, to using DNA based investigations to predict a physical characteristic of a samples source, and more particularly, but not exclusively to techniques for investigating or predicting the hair colouring and / or other pigmentation characteristics of a DNA source.

In a variety of situations it is desirable to be able to obtain as much information as possible about the identity or source of a DNA sample. Such situations include analysis of crime scene samples where it is helpful to obtain details of the potential source of that sample with a view to tracing the sample's source and/or linking a sample from a possible source to the crime scene sample and/or discounting a link between a sample from a possible source and the crime scene sample.

Forensic science already uses a variety of such techniques, such as short tandem repeat variations, to compare the DNA characteristics of one sample with a sample from a known person. These techniques concern variations in the DNA on an individual basis, however. Additionally they do not allow any prediction to be made about the source of a DNA sample, for instance from a crime scene sample, a physical characteristic of the individual who generated the DNA sample. Frandberg P-A et al: Biochem, Biophys, Res, vol. 245/2, 1998, p490-492 provides some information on the MC1R gene and variations therein. Smith R. et al: J Invest Dermatol Volume 111/1, 1998, p119-122 provides some information on variants of the melanocortin 1 receptor in the Irish population. Database Biosis, accession number PREV 1996999249107, Valverde Paloma et al; "the Asp84Glu variant of the melanocortin 1 receptor (MC1R) is associated with melanoma", XP002188624 and Human Molecular Genetics, Volume 5, number 10, 1996, p1663-1666, ISSN0964-6906 provides information on variation of the MC1R gene and susceptibility to melanoma which may arise as a result. All of these documents relate to research conducted on genetic material obtained from known individuals whose physical characteristics were known. There is no suggestion in this or the other prior art that DNA from an unknown individual can be examined with a view to determined his /her hair colour or skin pigmentation.

According to a first aspect of the invention a method of obtaining information about a person's hair colour and/or other pigmentation characteristics, the person being the source of a DNA sample, the method comprising
analysing the sample, the analysis involving considering the identity of any variation at the one or more of the locations at which variations potentially occur, the one or more locations being locations implicated in contributing to a person's hair colour or other pigmentation, the one or more locations being in the melanocortin receptor gene and/or the regulatory region for the melanocortin receptor gene;
providing information about the hair colour and/or other pigmentation characteristics of a person from whom the sample arose based on the results of the analysis of the one or more locations and which variations potentially occur.

The first aspect of the invention may provide one or more of the following features, options, possibilities or the like.

The information provided will be the hair colour and/or other pigmentation characteristics of the person who was the source of the sample. The information may be the skin colour and / or eye colour of the person who is the source of the sample. The information may be provided as a range, for instance a range of skin colour.

The information and/or nature of the physical characteristic may be a level of pigmentation. The nature of the physical characteristic may be whether it is of a particular colour type, such as red, and/or may be whether it is not of a particular type, such as not red hair.

The source may be male or female. The source may be a suspect in a crime and/or a person linked to the scene of a crime and/or a person linked to an item implicated in a crime and/or linked to the scene of a crime.

The sample may be any DNA containing sample, such as a blood sample, a bodily fluid sample, skin sample, hair sample or the like. The sample may be taken from a location, such as a wall, floor, floor covering or the like, and/or from an item, such as furniture, an item of clothing or the like.

The one or more locations are in the melanocortin receptor gene including in regulatory region, which locus is established to have a role in the production and/or distribution and/or consumption of melanin or other pigments, particularly in the human body. The area of DNA may be an area which produces an expression product and/or an area which regulates production.

The actual location of the one or more locations may be determined through sequencing the whole or parts of the locus implicated in the variations possible for the physical characteristic. The actual locations may be determined through whole sequencing of the locus. The actual location of the one or more locations may be determined by considering at least some locations in the DNA sequence for a number of people which have one nature for the physical characteristic and comparing the variation at those at least some locations in the DNA sequence for a number of people not having that nature and/or having a different nature for that physical characteristic. The nature may be red hair for one group. The nature may be not having red hair for the other group. The nature may be having blonde hair and/or brown hair and/or black hair for the other group.

The actual locations may comprise a number of potential locations of variation. The number of potential locations giving variation may be more than 3, preferably more than 5, more preferably more than 7 and ideally more than 10.

The actual locations may be one or more of, from the Mc1r loci, 86insA(codon 29), D84E, R142H, R151C, Y1520ch, I155T, R160W, D294H, 537insC (codon 180). Preferably the locations include at least one of Y152Och or 86insA(codon 29). Preferably the locations include at least one of Y152Och or 86insA(codon 29) and one or more of D84E, R142H, R151C, I155T, R160W, D294H, 537insC (codon 180).

The actual locations may be one or more of, from the Mc1r locus:-

| polymorphism / codon | normal base | position of base | mutation |
|---|---|---|---|
| 29 | | 86 INSERT | A |
| 60 | G | 178 | T |
| 84 | C | 252 | A |
| 92 | G | 274 | A |
| 142 | G | 425 | A |
| 151 | C | 451 | T |
| 152 | C | 456 | A |
| 155 | T | 464 | C |
| 160 | C | 478 | G |
| 163 | G | 488 | A |
| 180 | | 537 INSERT | C |
| 294 | G | 880 | C |

The variation at the actual locations may be one or more of:-

| polymorphism / codon and base location | normal amino acid | polymorphic amino acid |
|---|---|---|
| 29 86 | | |
| 60 178 | valine | leucine |
| 84 252 | aspartic acid | glutamic acid |
| 92 274 | valine | methionine |
| 142 425 | arginine | histidine |
| 151 451 | arginine | cysteine |
| 152 456 | tyrosine | STOP |
| 155 464 | isoleucine | threonine |
| 160 478 | arginine | tryptophan |
| 163 488 | arginine | glutamine |
| 180 537 | | |
| 294 880 | aspartic acid | histidine |

The actual locations are preferably not, from the Mc1r loci, V60L, V92M or R163Q.

The variations are preferably polymorphisms. The polymorphisms may be a variation in the DNA sequence. The polymorphism may be a variation in the DNA sequence for one or more of the above mentioned codon location's 29, 142, 151, 152, 155, 160, 180, 294. The polymorphisms may be a variation in the amino acid for one or more of the above mentioned codon location's 84, 142, 151, 152, 155, 160, 294. The actual locations may be one or more locations from the Pseudo Homeobox-containing Gene.

The variations may be thought of as having a normal identity and a mutated identity, differences in the identity being implicated in different natures of the physical characteristic. The normal identity for any one of the above mentioned codons may be the normal sequence and/or normal amino acid as defined in table 1 below. The mutated identity for any one of the above mentioned codons may be the polymorphic sequence and/or polymorphic amino acid as defined in table 1 below.

The presence of a mutated identity at one or more given locations may give an implication that a particular nature for the physical characteristic applies for that sample. The implication may be a suggestion, a statistical certainty or a definitive opinion.

The sample may be analysed by sequencing part or all of the sample, for one or more areas, such as loci. The analysis may sequence all or part of the Mc1r locus. The technique may only involve sequencing those portions of the DNA which are close to a location of possible variation and / or only the site of possible variation. The analysis preferably determines the identity of the sequence and/or amino acid present at the locations of potential variation.

The information may be provided based on a comparison of the sample results with the potential variations for the one or more locations. An identity of a variation may be indicative of a particular nature. An identity of a variation at two or more locations is preferably considered indicative of a particular nature. A mutated identity may be indicative of a particular nature. A mutated identity at two or more locations is preferably considered indicative of a particular nature.

The information may be to suggest a physical characteristic that the person from whom the sample arose has and / or is highly disposed towards, for instance red hair. The information may be to suggest a physical characteristic that the person from whom the sample arose does not have or is not disposed towards having, for instance the person does not have red hair. The suggestion may be provided together with an indication of statistical certainty. The suggestion may be provided together with caveats, for instance relating to dyed hair, greying hair or hair loss.

The reliability and/or statistical significance of the information may depend on the number of variations suggesting a particular nature for the physical characteristic which are found. The presence of two variations suggesting a particular nature may be seen as more significant than the presence of one variation.

The information may be used to corroborate information obtained in other ways about the source of the sample, for instance to confirm an eye witness account of the sample's source. The information may be used to direct subsequent investigations aimed at determining the sample's source, for instance the selection of groups of people having the predicted nature of the physical characteristic, with potentially that group then being subjected to a DNA screening process. The information may be used with other information to form a description of the suspected source of the sample.

According to a second aspect of the present invention we provide the use of one or more polymorphisms in the DNA of the melanocortin receptor gene, the polymorphisms including at least one of the polymorphisms situated at Y1520ch or 86insA (codon 29), to obtaining information about a person's hair colour and/or other pigmentation characteristics based on the results of the analysis of these polymorphisms for a DNA sample, the person being the source of the DNA sample.

The polymorphisms may include both of these polymorphisms. The polymorphisms may include one or more of D84E, R142H, R151C, I155T, R160W, D294H or537insC (codon 180).

Various embodiments of the invention will now be described, by way of example only, and with reference to the accompanying drawings in which :-
Figure 1 shows mini-sequencing profiles of the Mc1r locus for an individual with no polymorphisms within the gene, the sample being obtained from a dark haired individual.
Figure 2 is the mini-sequencing profile for the Mc1r locus for an individual who has the nucleotide polymorphism codon 151 on both chromosomes.

In a variety of situations a DNA sample may be obtained without definitive evidence as to its source. The tracing of that source and/or the confirmation or rebuttal of an entity as being the source is a significant forensic tool.

A number of existing techniques consider a variety of features of the DNA of a sample and compare that with features in a sample from a known source to establish whether the sample arose form that source and the statistical confidence in reaching that conclusion. Such techniques do not provide much information about the source of the sample, however, before such a comparison is made.

In the technique of the present invention, however, analysis of a sample is used to determine a likely physical characteristic of the source of the sample. These characteristics can then be used to assist in identifying groups of the population as a whole for particular investigation and/or be used alongside other evidence to assist in the tracing of the source of the sample.

The techniques described herein generally involve two stages, firstly the identification of variations implicated in the nature of a physical characteristic of an individual, secondly the use of a test for such variations to suggest the nature of a physical characteristic of an unknown individual.

In the first part of such a process, a target genetic area which is believed to have a role in the physical characteristic in question, and for which as a result variations in that area might cause the different natures of that physical characteristic, is considered. The investigative technique uses full sequencing and/or a mini sequencing techniques (see Tully et al., 1996 Genomics, 34:107-113) to consider the area (such as genes or parts thereof, for instance loci) under consideration to identify variations and consider those variations implication in variations in the physical characteristic under review.

As an example, the melanocortin receptor-1 (Mclr) is expressed in high levels in melanocytes and thus has a role in pigmentation. This locus was, therefore, considered as to whether or not variations within it were implicated in whether or not a person has red hair.

The investigative technique involved obtaining DNA samples from 64 red-headed persons and 150 non-red headed persons and performing a mutation spectrum consideration of those samples in relation to the Mc1r locus.

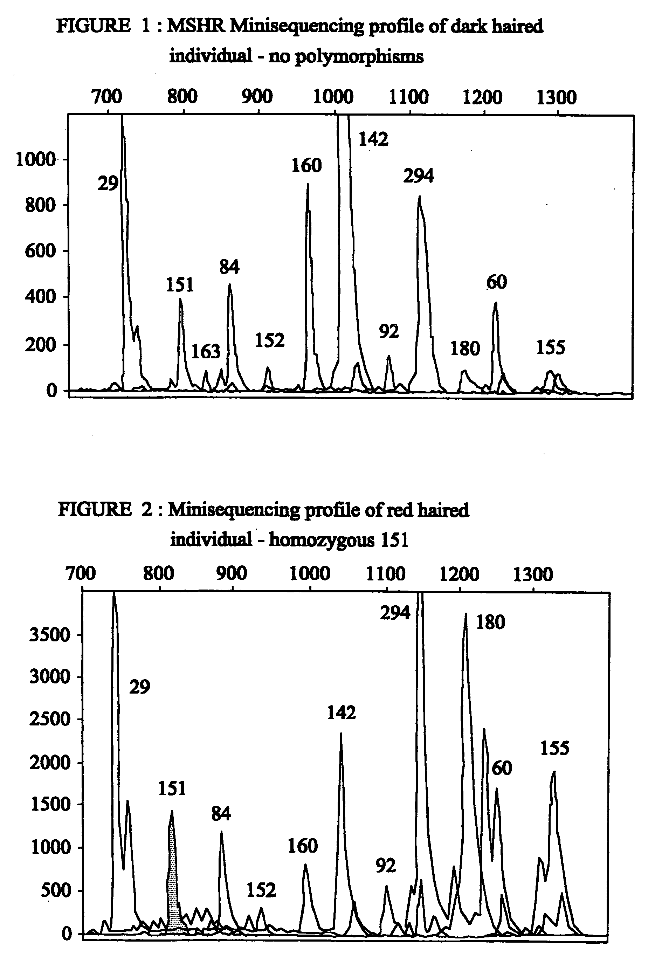

An example of the results for a dark haired person in the applicable area are set out in Figure 1. An example of the results for a red haired person are shown in Figure 2.

In the results, for instance, a single nucleotide mutation is demonstrated by a change in the peak colour for the profile, for instance from blue to green. Where overlapping blue and green peaks are noted, the individual would be classified as a heterozygote for that particular polymorphisms. A green dye may be used to indicate an A base, blue to indicate a G, red to indicate a C and yellow / black to indicate a T.

In the particular example of Figure 2, the individual exhibits of polymorphism as codon 151 on both chromosomes, thereby making the individual homozygous for the codon 151 polymorphism under consideration.

The results of the full sequencing of the locus indicate that there are 14 polymorphisms in this area. Of these, three, V60L, V92M and R163Q, where established as homozygous in several non-red haired persons and as a consequence not implicated in a person having red hair. Two of the polymorphisms do not cause a change in the amino acid and would therefore not result in an altered gene product and affect hair colour or other pigmentation characteristics. The silent polymorphisms are codon 274 (G274G) = C822G causing glycine to remain as glycine and codon 314 (T314T) = A942G causing threonine to remain as threonine. Of the others, nine are believed to be implicated in a person having red hair as in 92% of homozygotes and compound heterozygotes loss of function arose from these mutations. The polymorphisms are summarised in Table 1. The early termination mutation, Y1520ch, and the frame shift mutation, 86insA (codon 29) which results in premature termination twelve amino acids later, are particularly significant. It should be noted that the nucleotide designations given in Table 1 all refer to those seen on the same DNA strand. As analysis involves consideration of both strands due to the techniques used, the variation in the complimentary strand will be detected.

**Table 1.**

| Codon | normal sequence | normal amino acid | polymorphic sequence | polymorphic amino acid |
|---|---|---|---|---|
| 29 | aac | | aaa | |
| 60 | gtg | valine | ttg | leucine |
| 84 | gac | aspartic acid | gaa | glutamic acid |
| 92 | gtg | valine | atg | methionine |
| 142 | cgc | arginine | cac | histidine |
| 151 | cgc | arginine | tgc | cysteine |
| 152 | tac | tyrosine | taa | STOP |
| 155 | atc | isoleucine | acc | threonine |
| 160 | cgg | arginine | tgg | tryptophan |
| 163 | gag | arginine | aag | glutamine |
| 180 | atc | | cat | |
| 294 | gac | aspartic acid | cac | histidine |

Of the red haired persons tested, 80% had two or more of the different red hair implicated mutations. Of the others, 11 had only one of the mutations and 2 had none, and red hair may be caused in these cases by mutations elsewhere in the melanin biosynthesis pathway.

Only 2 of the non red headed persons were found to possess two of the different red hair implicated mutations of the Mc1r locus. It is believed that these two individuals had the mutations on the same chromosome, and that checking the phasing of mutations, and that checking this can be used to verify whether a two or more mutation result is implicating in red hair or not for an unknown sample. The phasing of mutations is checked by determining the chromosomal location of the polymorphisms. Polymorphisms on both chromosomes are more likely to result in the particular nature of the physical characteristic, as both polymorphisms occurring on the same chromosome will still result in one normally functioning copy of the gene.

Once the sites of the potential variations in the target sequence have been identified the technique can be used to predict physical characteristic for the sources of unknown samples.

The sample investigation part of the process thus involves the collection of a DNA sample from a crime scene, or the like, in the conventional way for subsequent analysis. The analysis itself uses the mini sequencing process referred to above to reveal the sequence of the sample DNA at the locations of interest. The results may show that the sample of unknown origin possesses two or more of the mutations implicated in a particular nature for the physical characteristic under review. More specifically the presence of two or more of the mutations summarised in Table 1 (other than those for codons 60, 92 and 163) may be taken as an indicator of source of the sample having red hair. The presence of only one of the mutations may provide a less clear suggestion that the person does have red hair and the absence of any of the mutations may provide a strong indication that the person does not have red hair.

The outcome of the sample investigation stage can be used to corroborate other evidence relating to a matter, for instance that a suspect has red hair in keeping with eye witness accounts. The outcome of the sample investigation could be used to determine the strategy for further enquires in to the matter, for instance the determination of a grouping of individuals (having the suggested red hair, for instance) for DNA screening and/or other investigations.

Whilst the technique is described above in relation to investigations of the Mc1r loci including the regulatory region for Mc1r, other locations could be considered for red hair implication and/or other pigment influences and/or influence on other physical characteristic. The consideration of the other genes effecting Mc1r expression and/or other genes acting within and/or outside the Mc1r signalling pathway may be pursued.

## Claims

1. A method of obtaining information about a person's hair colour and/or other pigmentation characteristics, the person being the source of a DNA sample, the method comprising
analysing the sample, the analysis involving considering the identity of any variation at the one or more of the locations at which variations potentially occur, the one or more locations being locations implicated in contributing to a person's hair colour or other pigmentation, the one or more locations being in the melanocortin receptor gene and/or the regulatory region for the melanocortin receptor gene;
providing information about the hair colour and/or other pigmentation characteristics of a person from whom the sample arose based on the results of the analysis of the one or more locations and which variations potentially occur.

2. A method according to claim 1 in which the sample is a blood sample, a bodily fluid sample, a skin sample or a hair sample.

3. A method according to claim 1 or claim 2 in which the actual locations are one or more of, from the melanocortin receptor gene, 86insA (codon 29), D84E, R142H, R151C, Y152Och, I155T, R160W, D294H, 537insC (codon 180).

4. A method according to any of claims 1 to 3 in which the variations are thought of as having a normal identity and a mutated identity, differences in the identity being implicated in different natures of the characteristic.

5. A method according to any of claims 1 to 4 in which the presence of a mutated identity at one or more given locations gives an implication that a particular nature for the characteristic applies for that sample.

6. A method according to any of claims 1 to 5 in which the information provides a level of pigmentation for the person.

7. A method according to any of claims 1 to 6 in which the information suggests a characteristic that the person from whom the sample arose has and/or is highly disposed towards.

8. A method according to any of claims 1 to 7 in which the information suggests the person has red hair.

9. A method according to any of claims 1 to 8 in which the information suggests a characteristic that the person from whom the sample arose does not have and/or is not disposed towards having.

10. A method according to any of claims 1 to 7 or claim 9 in which the information suggests the person does not have red hair.

11. A method according to any of claims 1 to 10 in which the suggestion is provided together with an indication of statistical certainty.

12. A method according to any of claims 1 to 11 in which the information is used to assist in the tracing of the person who is the source of the sample.

13. The use of one or more polymorphisms in the DNA of the melanocortin receptor gene, the polymorphisms including at least one of the polymorphisms situated at Y1520ch or 86insA (codon 29), to obtaining information about a person's hair colour and/or other pigmentation characteristics based on the results of the analysis of these polymorphisms for a DNA sample, the person being the source of the DNA sample.

14. The use of claim 13 in which the polymorphisms include one or more of D84E, R142H, R151C, I155T, R160W, D294H, 537insC (codon 180).

## Patentansprüche

1. Verfahren zum Erhalten von Informationen über Haarfarbe und/oder andere Pigmentierungscharakteristika einer Person, wobei die Person die Quelle einer DNA-Probe ist, wobei das Verfahren umfasst:
- Analysieren der Probe, wobei die Analyse Betrachten der Identität einer Variation an einer Stelle oder mehreren Stellen, an denen potentiell Variationen auftreten, involviert, wobei die eine Stelle oder mehreren Stellen Stellen sind, die impliziert sind, einen Beitrag zur Haarfarbe oder zu einer anderen Pigmentierung einer Person zu leisten, wobei die eine Stelle oder die mehreren Stellen im Melanocortinrezeptorgen und/oder der regulatorischen Region für das Melanocortinrezeptorgen ist/sind;
- Bereitstellen von Informationen über die Haarfarbe und/oder andere Pigmentierungscharakteristika einer Person, aus der die Probe stammt, auf der Basis der Resultate der Analyse der einen Stelle oder der mehreren Stellen und welche Variationen potentiell auftreten.

2. Verfahren nach Anspruch 1, wobei die Probe eine Blutprobe, eine Körperflüssigkeitsprobe, eine Hautprobe oder eine Haarprobe ist.

3. Verfahren nach Anspruch 1 oder Anspruch 2, wobei die tatsächlichen Stellen eine oder mehrere der folgenden aus dem Melanocortinrezeptorgen 86insA (Codon 29), D84E, R142H, R151C, Y1520ch, I155T, R160W, D294H, 537insC (Codon 180) ist/sind.

4. Verfahren nach einem der Ansprüche 1 bis 3, wobei die Variationen als solche mit einer normalen Identität und einer mutierten Identität angenommen werden, wobei Unterschiede in der Identität in verschiedenen Naturen des Charakteristikums impliziert sind.

5. Verfahren nach einem der Ansprüche 1 bis 4, wobei das Vorliegen einer mutierten Identität an einer gegebenen Stelle oder mehreren gegebenen Stellen eine Implikation liefert, dass eine bestimmte Natur für das Charakteristikum für jene Probe zutrifft.

6. Verfahren nach einem der Ansprüche 1 bis 5, wobei die Informationen einen Pigmentierungslevel für die Person bereitstellen.

7. Verfahren nach einem der Ansprüche 1 bis 6, wobei die Informationen einen Hinweis auf ein Charakteristikum geben, das die Person, von der die Probe stammt, hat und/oder wozu sie stark neigt.

8. Verfahren nach einem der Ansprüche 1 bis 7, wobei die Informationen einen Hinweis geben, dass die Person rotes Haar hat.

9. Verfahren nach einem der Ansprüche 1 bis 8, wobei die Informationen einen Hinweis geben, dass die Person, von der die Probe stammt, ein Charakteristikum nicht hat und/oder nicht dazu neigt, es zu haben.

10. Verfahren nach einem der Ansprüche 1 bis 7 oder Anspruch 9, wobei die Informationen einen Hinweis geben, dass die Person kein rotes Haar hat.

11. Verfahren nach einem der Ansprüche 1 bis 10, wobei der Hinweis zusammen mit einem Hinweis für statistische Sicherheit bereitgestellt wird.

12. Verfahren nach einem der Ansprüche 1 bis 11, wobei die Informationen verwendet werden, um die Nachforschungen (tracing) (bezüglich) der Person, die die Quelle der Probe ist, zu unterstützen.

13. Die Verwendung eines Polymorphismus oder mehrerer Polymorphismen in der DNA des Melanocortinrezeptorgens, wobei die Polymorphismen wenigstens einen der Polymorphismen einschließen, die an Y1520ch oder 86insA (Codon 29) liegen, zum Erhalten von Informationen über Haarfarbe und/oder andere Pigmentierungscharakteristika einer Person auf der Basis der Resultate der Analyse dieser Polymorphismen für eine DNA-Probe, wobei die Person die Quelle der DNA-Probe ist.

14. Verwendung nach Anspruch 13, wobei die Polymorphismen einen oder mehrere von D84E, R142H, R151C, I155T, R160W, D294H, 537insC (Codon 180) umfassen.

## Revendications

1. Procédé pour obtenir des informations sur la couleur des cheveux et/ou sur d'autres caractéristiques de pigmentation d'une personne, cette personne étant la source d'un échantillon d'ADN, le procédé comprenant les étapes consistant à
analyser l'échantillon, cette analyse impliquant de considérer l'identité d'une variation quelconque au niveau d'un ou plusieurs des emplacements auxquels des variations peuvent survenir, les un ou plusieurs emplacements étant des emplacements impliqués dans la contribution à la couleur des cheveux d'une personne ou à une autre pigmentation, les un ou plusieurs emplacements étant dans le gène du récepteur de la mélanocortine et/ou dans la région régulatrice du gène du récepteur de la mélanocortine ;
fournir des informations sur la couleur des cheveux et/ou d'autres caractéristiques de pigmentation d'une personne dont l'échantillon provient en se fondant sur les résultats de l'analyse des un ou plusieurs emplacements et sur les variations qui peuvent survenir.

2. Procédé selon la revendication 1 dans lequel l'échantillon est un échantillon de sang, un échantillon de liquide organique, un échantillon de peau ou un échantillon de cheveux.

3. Procédé selon la revendication 1 ou la revendication 2 dans lequel les emplacements réels sont un ou plusieurs, dans le gène du récepteur de la mélanocortine, de 86insA (codon 29, D84E, R142H, R151C, Y152Och, 1155T, R160W, D294H, 537insC (codon 1980).

4. Procédé selon l'une quelconque des revendications 1 à 3 dans lequel les variations sont envisagées comme ayant une identité normale et une identité mutée, des différences d'identité étant impliquées dans différentes natures de la caractéristique.

5. Procédé selon l'une quelconque des revendications 1 à 4 dans lequel la présence d'une identité mutée au niveau d'un ou plusieurs emplacements donnés donne une implication qu'une nature particulière de la caractéristique s'applique à cet échantillon.

6. Procédé selon l'une quelconque des revendications 1 à 5 dans lequel les informations fournissent un niveau de pigmentation pour la personne.

7. Procédé selon l'une quelconque des revendications 1 à 6 dans lequel les informations suggèrent une caractéristique que la personne dont provient l'échantillon présente et/ou est fortement disposée à présenter.

8. Procédé selon l'une quelconque des revendications 1 à 7 dans lequel les informations suggèrent que la personne a les cheveux roux.

9. Procédé selon l'une quelconque des revendications 1 à 8 dans lequel les informations suggèrent une caractéristique que la personne dont provient l'échantillon ne présente pas et/ou n'est pas disposée à présenter.

10. Procédé selon l'une quelconque des revendications 1 à 7 ou 9 dans lequel les informations suggèrent que la personne n'a pas les cheveux roux.

11. Procédé selon l'une quelconque des revendications 1 à 10 dans lequel la suggestion est fournie avec une indication de certitude statistique.

12. Procédé selon l'une quelconque des revendications 1 à 11 dans lequel les informations sont utilisées pour aider à retrouver la personne qui est la source de l'échantillon.

13. Utilisation des un ou plusieurs polymorphismes de l'ADN du gène du récepteur de la mélanocortine, les polymorphismes incluant au moins l'un des polymorphismes situés sur Y1520ch ou 86insA (codon 29), pour obtenir des informations sur la couleur des cheveux et/ou d'autres caractéristiques de pigmentation d'une personne en se fondant sur les résultats de l'analyse de ces polymorphismes pour un échantillon d'ADN, la personne étant la source de l'échantillon d'ADN.

14. Utilisation selon la revendication 13 dans laquelle les polymorphismes incluent un ou plusieurs parmi D84E, R142H, R151C, I155T, R160W, D294H, 537insC (codon 180).
